(19) Europäisches Patentamt
European Patent Office
Office européen des brevets

(11) **EP 3 023 062 A1**

(12) **EUROPEAN PATENT APPLICATION**

(43) Date of publication:
**25.05.2016 Bulletin 2016/21**

(51) Int Cl.:
**A61B 17/135** (2006.01)    **A61H 9/00** (2006.01)
**A61B 5/021** (2006.01)    **A61B 5/0225** (2006.01)

(21) Application number: **15194463.4**

(22) Date of filing: **13.11.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **19.11.2014  JP 2014234778**

(71) Applicant: **Nihon Kohden Corporation Shinjuku-ku Tokyo (JP)**

(72) Inventor: **TANAKA, Rie Shinjuku-ku, Tokyo (JP)**

(74) Representative: **Grünecker Patent- und Rechtsanwälte PartG mbB Leopoldstraße 4 80802 München (DE)**

(54) **COMPRESSION CONTROL DEVICE AND COMPRESSION CONTROL METHOD**

(57)    A compression control device includes a cuff that is adapted to be wrapped to a first region of a living body, a pressure controlling section that controls an application pressure to be applied to the first region by the cuff, and a blood pressure obtaining section that obtains a blood pressure value from the living body. The pressure controlling section controls the application pressure based on the blood pressure value obtained by the blood pressure obtaining section in a state where a prescribed pressure for causing an ischemic state in a part from the first region to a periphery is applied to the first region by the cuff.

FIG. 1

EP 3 023 062 A1

**Description**

BACKGROUND

The present invention relates to a compression control device and a compression control method for compressing an upper limb, a lower limb or the like of a patient.

There is a conventional procedure employed in an orthopedic surgery of an upper limb or a lower limb for reducing the amount of bleeding in a surgical field by compressing, with an air pressure, an upper arm or a thigh by using a compression bandage such as a tourniquet wound therearound so as to throttle blood flow to the periphery.

Besides, a procedure designated as remote ischemic preconditioning has been recently proposed. This procedure is performed, before performing a surgery or the like in which ischemic and reperfusion injury is presumed to occur, for purpose of improving ischemia resistance of an organ to be treated by causing ischemia and reperfusion of a short period of time several times in a region away from the heart.

In both the procedure for reducing the amount of bleeding and the remote ischemic preconditioning, a treatment for causing an ischemic state in a part of a patient's body from a region such as an upper arm or a thigh to a periphery is significant.

For example, Japanese Patent No. 5323719 discloses a system for performing the remove ischemic preconditioning on a patient.

In the system described in Japanese Patent No. 5323719, however, although an ischemic state is caused with a prescribed pressure, the ischemic state of a peripheral part of the patient's body cannot be appropriately retained in some cases because the blood pressure value is varied.

Accordingly, an object of the present invention is to provide a compression control device and a compression control method by which an ischemic state of a peripheral part of a patient's body can be appropriately retained.

SUMMARY

A compression control device includes a cuff adapted to be wrapped around a first region of a living body, a pressure controlling section that controls an application pressure to be applied to the first region by the cuff, and a blood pressure obtaining section that obtains a blood pressure value from the living body. The pressure controlling section controls the application pressure based on the blood pressure value obtained by the blood pressure obtaining section in a state where a prescribed pressure for causing an ischemic state in a part from the first region to a periphery is applied to the first region by the cuff.

BRIEF DESCRIPTION OF DRAWINGS

Fig. 1 is a schematic diagram of a bedside monitor according to a first embodiment of the present invention

Fig. 2 is a flowchart for illustrating an operation of the bedside monitor of Fig. 1.

Fig. 3 is a diagram illustrating an example of an estimated blood pressure waveform of a patient and a cuff pressure waveform in remote ischemic preconditioning.

Fig. 4 is a schematic diagram of a bedside monitor according to a second embodiment of the present invention

Fig. 5 is a flowchart for illustrating an operation of the bedside monitor of Fig. 4.

Fig. 6 is a diagram for illustrating a modification in which a compression pattern employed in the bedside monitor is changed.

Fig. 7 is a diagram illustrating a waveform of a cuff pressure applied in conventional remote ischemic preconditioning.

DETAILED DESCRIPTION OF EMBODIMENTS

An exemplified embodiment of the present invention will now be described in detail with reference to the accompanying drawings. As an example of a device according to the present embodiment, a bedside monitor 1 having a compression control function will now be described.

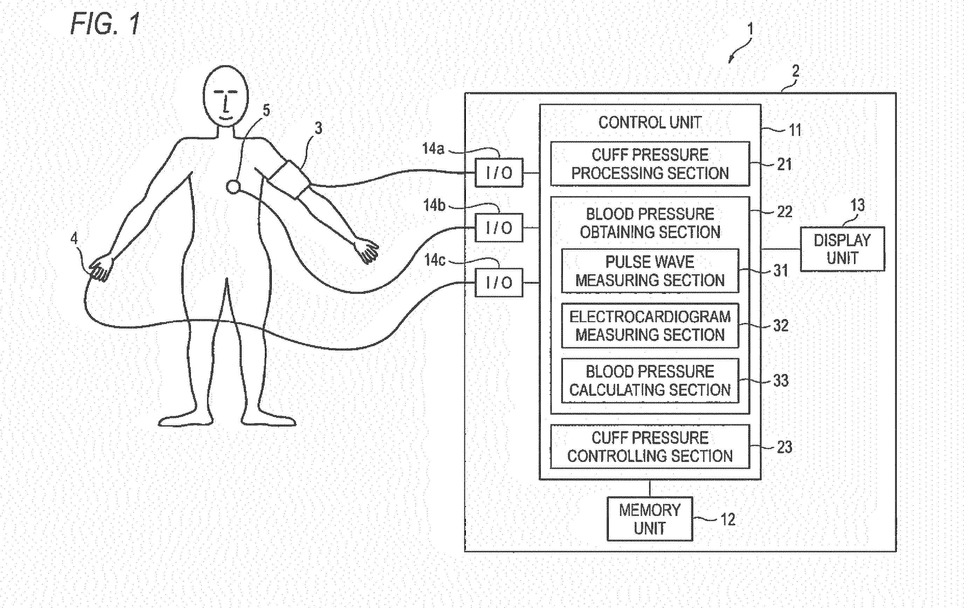

As shown in Fig. 1, the bedside monitor 1 of the present embodiment can include a main body part 2 having a control unit 11, a memory unit 12, a display unit 13 and input/output ports 14a to 14c. A cuff 3 is connected to the bedside monitor 1 via the input/output port 14a. Besides, an electrode 5 for obtaining an electrocardiogram is connected to the bedside monitor 1 via the input/output port 14b. Furthermore, a probe 4 for obtaining vital sign such as a pulse wave or $SpO_2$ is connected to the bedside monitor 1 via the input/output port 14c.

The cuff 3 is wrapped to a part of a patient's body (corresponding to an example of a living body) where a vein and an artery of the living body can be detected, such as a left upper arm (corresponding to an example of a first region). The cuff 3 includes an air bag (not shown), and when the air is fed to the air bag, a cuff pressure (an application pressure), that is, a pressure of the cuff 3, is applied to the upper arm.

The probe 4 is attached to a part of the living body where a vein or an artery of the patient can be detected, such as a fingertip that is a peripheral region of the right hand (corresponding to an example of a second region). The probe 4 is attached to a region different from a region where the cuff 3 is wrapped. Alternatively, the probe may be attached to a toe of a foot. Besides, the electrode 5 is attached to the patient's chest.

The control unit 11 controls the operations of the respective units of the bedside monitor 1. The control unit 11 can include a cuff pressure processing section 21, a blood pressure obtaining section 22 and a cuff pressure controlling section (corresponding to an example of a pressure controlling section) 23. The control unit 11 has functions as these sections.

The cuff pressure processing section 21 can measure, based on a pressure waveform obtained by the cuff 3, a systolic blood pressure value (a maximum blood pressure value), a mean blood pressure value, a diastolic blood pressure value (a minimal blood pressure value) and the like.

The blood pressure obtaining section 22 non-invasively obtains a blood pressure value of the patient based on the vital sign obtained by the probe 4 and the electrode 5. The blood pressure obtaining section 22 can include a pulse wave measuring section 31, an electrocardiogram measuring section 32 and a blood pressure calculating section (corresponding to an example of a calculating section) 33.

The pulse wave measuring section 31 measures a peripheral pulse wave of the patient based on the vital sign obtained by the probe 4.

The electrocardiogram measuring section 32 measures an electrocardiogram of the patient based on the vital sign obtained by the electrode 5.

The blood pressure calculating section 33 calculates a PWTT (Pulse Wave Transit Time) based on the peripheral pulse wave measured by the pulse wave measuring section 31 and the electrocardiogram measured by the electrocardiogram measuring section 32. The PWTT corresponds to a time elapsing from an R wave of the electrocardiogram to the appearance of a peripheral pulse wave, and is obtained by detecting a peak of the R wave and a bottom peak (a rising point) of the peripheral pulse wave, and is calculated based on a difference therebetween. The blood pressure calculating section 33 calculates, based on the calculated PWTT, an estimated blood pressure value, that is, a maximum blood pressure value estimated for the patient. A specific calculation method will be described later.

The cuff pressure controlling section 23 controls, based on the estimated blood pressure value calculated by the blood pressure calculating section 33, a cuff pressure to be applied by the cuff 3 to the left upper arm of the patient.

The memory unit 12 stores the vital sign obtained from the patient, data measured or calculated by the respective sections of the control unit 11, and the like.

The display unit 13 includes, for example, a touch panel-type liquid crystal screen, and displays various information obtained by the control unit 11.

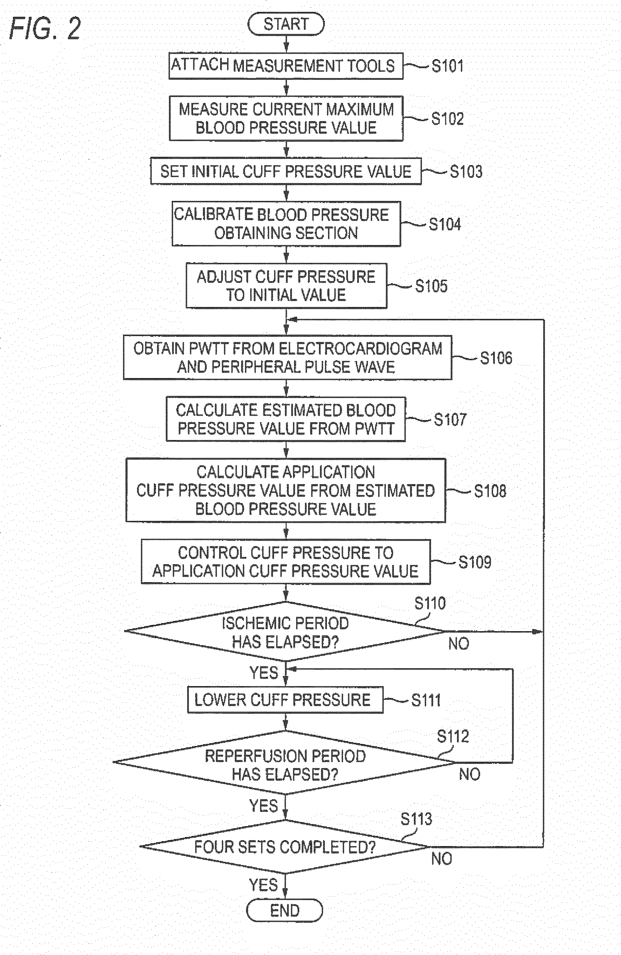

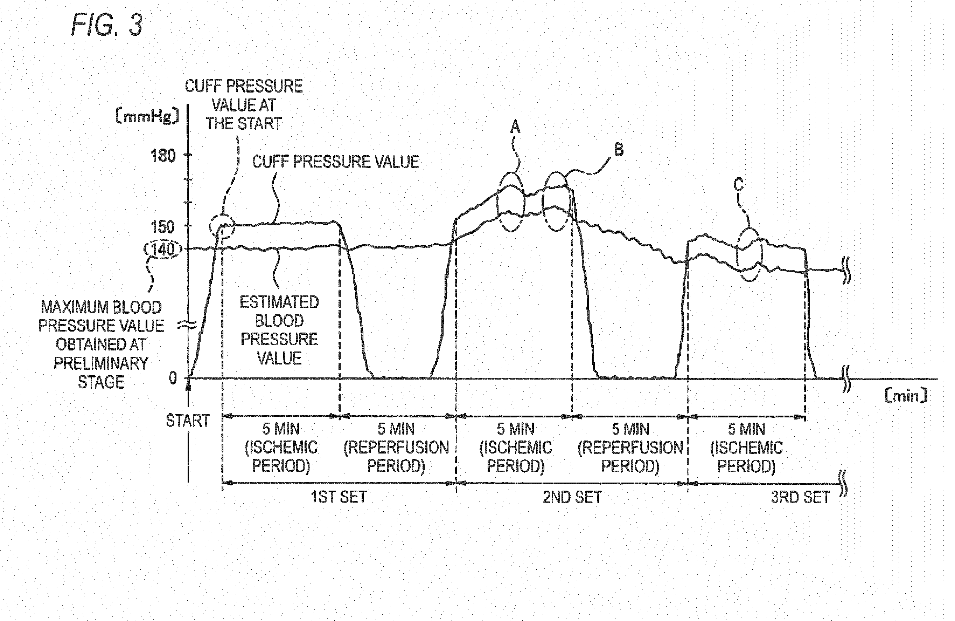

Next, with reference to Figs. 2 and 3, a compression control method performed in the bedside monitor 1 will be described. This embodiment describes a case where the remote ischemic preconditioning is performed on a patient. It is assumed that a region of the patient's body in which an ischemic state and a reperfusion state are caused in the remote ischemic preconditioning is a peripheral region distal to the left upper arm on which the cuff 3 is wrapped as illustrated in Fig. 1.

(Preliminary stage of remote ischemic preconditioning)

First, measurement tools such as the cuff 3, the probe 4 and the electrode 5 are attached to the patient (step S101).

Next, based on a pressure waveform obtained by the cuff 3, the cuff pressure processing section 21 measures a current maximum blood pressure value of the patient at the preliminary stage (step S102). In this embodiment, it is assumed that the maximum blood pressure value at the preliminary stage is 140 mmHg.

Subsequently, the blood pressure calculating section 33 determines a value of a cuff pressure (corresponding to an example of an initial target value) to be applied at the start of the remote ischemic preconditioning (step S103). This value of the cuff pressure is determined as a value (150 mmHg) higher than the maximum blood pressure value (140 mmHg) measured in step S102 by, for example, 10 mmHg.

The value of the cuff pressure to be applied is determined as a prescribed pressure value necessary for causing an ischemic state. The prescribed pressure value refers to a pressure value higher than the maximum blood pressure value of the patient by a precedently determined pressure value. The precedently determined pressure value may be arbitrarily selected from, for example, 5 mmHg, 10 mmHg, 15 mmHg, 20 mmHg, 25 mmHg, 30 mmHg and the like. Alternatively, the prescribed pressure value may be set to any pressure ranging from 103% to 120% of the maximum blood pressure value.

Subsequently, in order to calculate an estimated blood pressure value based on the pulse wave obtained from the probe 4 and the electrocardiogram obtained from the electrode 5, the blood pressure obtaining section 22 is calibrated (step S104). The calibration is performed as follows in accordance with a relational expression between a maximum blood pressure value and a pulse wave transit time.

Assuming that the maximum blood pressure value is P, the maximum blood pressure value (P) is expressed, for example, by the following expression 1:

$$P = \alpha \cdot PWTT + \beta \quad \text{...... (Expression 1)}$$

wherein PWTT is the pulse wave transit time, $\alpha$ is an inherent coefficient, and $\beta$ is an inherent value of each patient.

Here, in order to obtain the inherent value $\beta$ of the patient, the maximum blood pressure value and the pulse wave transit time are measured. The maximum blood pressure value is measured by using the cuff 3. The pulse wave transit time is measured by using the blood pressure calculating section 33.

Assuming that the maximum blood pressure value is P1 and that the pulse wave transit time is PWTTI, the relationship therebetween is expressed by the following expression 2:

$$PI = \alpha \cdot PWTTI + \beta \quad ... \quad (Expression\ 2)$$

The inherent value $\beta$ of the patient is precedently obtained by substituting actually measured values in these expressions.

(Remote ischemic preconditioning)

After completing the above-described preliminary stage, the remote ischemic preconditioning is started. An ischemia time, a reperfusion time and the number of cycles can be set. In this embodiment, description will be given on the assumption that a time period of an ischemic state and a time period of a reperfusion state retained in the remote ischemic preconditioning are respectively set to 5 minutes, and that a cycle of a combination of the ischemic period of 5 minutes and the reperfusion period of 5 minutes defined as one set is repeated by four times, namely, as four sets.

First, the cuff pressure controlling section 23 increases the cuff pressure to be applied to the left upper arm of the patient up to 150 mmHg, that is, the initial cuff pressure value determined in step S103 (step S105).

Referring to Fig. 3, the cuff pressure at this time point is increased from a starting point of the first set to reach 150 mmHg, that is, the initial cuff pressure value. Since the pressure higher, by 10 mmHg, than the maximum blood pressure value (140 mmHg) obtained at the preliminary stage is applied by the cuff 3, an ischemic state is started to be caused in the peripheral region distal to the left upper arm of the patient.

Then, in the same manner as in the measurement performed in step S104, the pulse wave transit time of the patient is measured based on the electrocardiogram and the peripheral pulse wave (step S106).

Subsequently, based on the pulse wave transit time (PWTTn) measured in step S106, the blood pressure calculating section 33 non-invasively calculates a current estimated blood pressure value of the patient (step S107).

Assuming that the estimated blood pressure value is EBP, the estimated blood pressure value (EBP) is expressed by the following expression 3:

$$EBP = \alpha \cdot PWTTn + \beta \quad ... \quad (Expression\ 3)$$

As the value $\beta$, the inherent value of the patient obtained in step S104 is used.

Next, based on the estimated blood pressure value obtained in step S107, the blood pressure calculating section 33 calculates a value of the cuff pressure to be applied by the cuff 3 (step S108). The value of the cuff pressure is set to a value higher, by 10 mmHg, than the estimated blood pressure value (EBP). The calculated value of the cuff pressure is fed back from the blood pressure calculating section 33 to the cuff pressure controlling section 23 as data for controlling the pressure of the cuff 3.

Next, with the cuff pressure for causing the ischemic state applied to the left upper arm of the patient, the cuff pressure controlling section 23 controls the cuff pressure to be applied to the left upper arm by the cuff 3 to the value of the cuff pressure calculated in step S108 (step S109).

Then, it is determined whether or not 5 minutes duration of the ischemic state has elapsed (step S110). If it is determined that 5 minutes duration have not elapsed, the processing returns to step S106. If it is determined that 5 minutes duration have elapsed, the ischemic period of the current set is completed, and the processing proceeds to step S111.

Here, referring to Fig. 3, the estimated blood pressure value of the patient is stabilized at approximately 140 mmHg during the ischemic period of the first set. Therefore, the cuff pressure value is controlled to be kept at approximately 150 mmHg, which is higher by 10 mmHg than the estimated blood pressure value. Besides, in the ischemic period of the second set, the estimated blood pressure value is varied to increase beyond the maximum blood pressure value obtained at the preliminary stage. Therefore, the cuff pressure value is controlled to be higher, by 10 mmHg, than the increased estimated blood pressure value. Besides, in the ischemic period of the third set, the estimated blood pressure value is lowered below the maximum blood pressure value obtained at the preliminary stage. Therefore, the cuff pressure value is controlled to be higher, by 10 mmHg, than the lowered estimated blood pressure value. In this manner, in the ischemic period of any set, the cuff pressure value is controlled so as to appropriately retain the ischemic state.

In step S111 after the elapse of the ischemic period, the cuff pressure controlling section 23 discharges the air fed to the air bag of the cuff 3 to lower the cuff pressure. When the cuff pressure is lowered below the minimal blood pressure value (the cuff pressure is lowered to be substantially 0 mmHg in this embodiment), the peripheral region distal to the left upper arm is released to be in a reperfusion state.

Subsequently, it is determined that whether or not 5 minutes duration of the reperfusion state has elapsed (step S112). If it is determined that 5 minutes duration have not elapsed, the processing returns to step S111. If it is determined that 5 minutes duration have elapsed, the reperfusion period of the current set is completed, and the processing proceeds to step S113.

In step S113, it is determined whether or not the four sets of the cycle of the ischemic period of 5 minutes duration and the reperfusion period of 5 minutes duration have completed after starting the ischemic preconditioning. If it is determined that the four sets have not been completed, the processing returns to step S106. If it is determined that the four sets have been completed, the processing of the remote ischemic preconditioning is terminated.

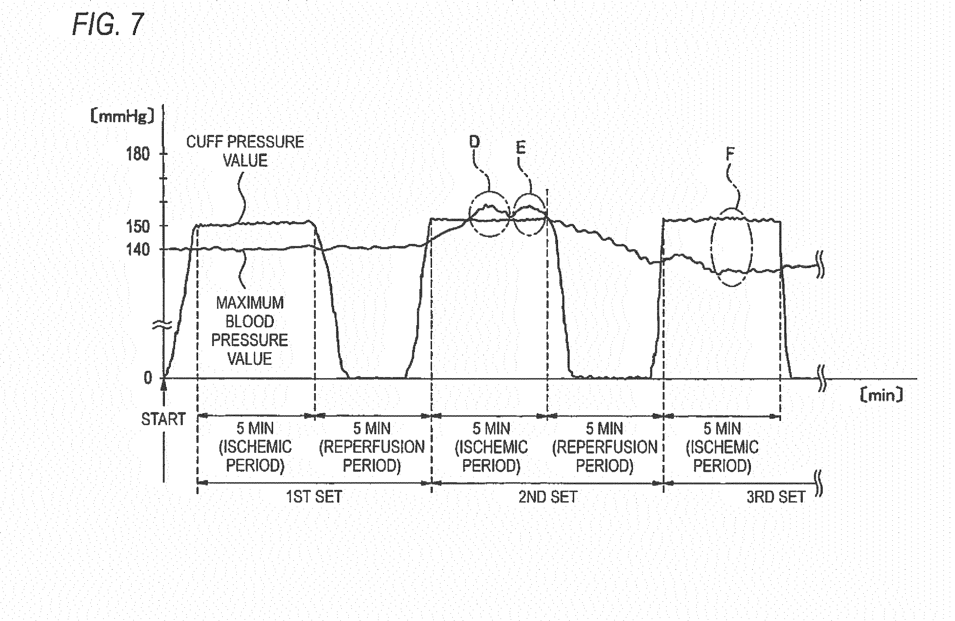

In the conventional remote ischemic preconditioning, if an ischemic state is to be caused in a peripheral region away from the heart, such as an arm or a foot, a precedently determined constant cuff pressure (of 150 mmHg) is applied in each ischemic period, for example, as illustrated in Fig. 7.

In the exemplified case of Fig. 7, in the ischemic period of the first set, the maximum blood pressure value of the patient is not varied but stabilized at a substantially constant value of 140 mmHg, and the cuff pressure is also retained constant.

On the other hand, in the ischemic period of the second set, the maximum blood pressure value of the patient is often increased due to a factor of, for example, medication, ischemic stress or the like. For example, in regions D or E, the increased maximum blood pressure becomes higher than the cuff pressure value (of 150 mmHg), and hence, the ischemic state is not appropriately retained. Incidentally, the phenomenon that the ischemic state is not retained can occur even if the maximum blood pressure value does not exceed the cuff pressure value but becomes close to the cuff pressure value to some extent.

Besides, in the ischemic period of the third set, the maximum blood pressure value of the patient is lowered due to a factor of, for example, reduction of the amount of circulating blood caused by bleeding or dehydration, cardiac hypofunction or the like. For example, in a region F, a difference between the lowered maximum blood pressure value and the cuff pressure value (of 150 mmHg) is larger than the prescribed difference (of 10 mmHg). As a result, an excessive pressure beyond the cuff pressure necessary for retaining the ischemic state is applied to the patient, which increases the load on the patient. In this manner, also in the ischemic period of the third set, the ischemic state is not appropriately retained.

On the contrary, when the bedside monitor 1 of the present embodiment is used, a current estimated blood pressure value of a patient is continuously obtained and a cuff pressure value according with the obtained current estimated blood pressure value is continuously calculated during the remote ischemic preconditioning. Then, the calculated cuff pressure value is fed back to the cuff pressure controlling section 23, and the cuff pressure to be applied by the cuff 3 is controlled based on the estimated blood pressure value that can be continuously changed. Therefore, a cuff pressure suitable to the current blood pressure value of the patient can be applied.

Even if, for example, as in the ischemic period of the second set illustrated in Fig. 3, the estimated blood pressure value of the patient is increased to exceed, for example, the cuff pressure value (of 150 mmHg) obtained at the start as in regions A and B, the cuff pressure can be increased to a suitable pressure (of, for example, the estimated blood pressure value + 10 mmHg) at which the ischemic state is not released. Besides, even if the estimated blood pressure value of the patient is lowered, as in the ischemic period of the third set, to be lower than the cuff pressure value obtained at the start as in a region C, the cuff pressure can be lowered to a suitable pressure at which the ischemic state is not released and at which an excessive load is not applied to the left upper arm having the cuff 3 wrapped thereon. Accordingly, the cuff pressure applied by the cuff 3 can be appropriately controlled so as to follow the change of the blood pressure value of the patient during the procedure, and hence, the ischemic state of the peripheral region can be appropriately retained.

Besides, in the bedside monitor 1 of the present embodiment, the current vital sign of the patient is measured in the regions different from the left arm in which the ischemic state is caused by the cuff 3 (specifically, in the fingertip of the right hand and the chest), and a current estimated blood pressure value of the patient is obtained based on the vital sign. Therefore, a highly precise estimated blood pressure value minimally affected by the ischemic procedure can be obtained.

Furthermore, since the vital sign obtained by using the probe 4 and the electrode 5 is used, a current estimated blood pressure value of the patient can be non-invasively obtained.

Besides, the compression control performed in the remote ischemic preconditioning can be automatically executed. Therefore, the burden of a healthcare professional during the procedure can be reduced.

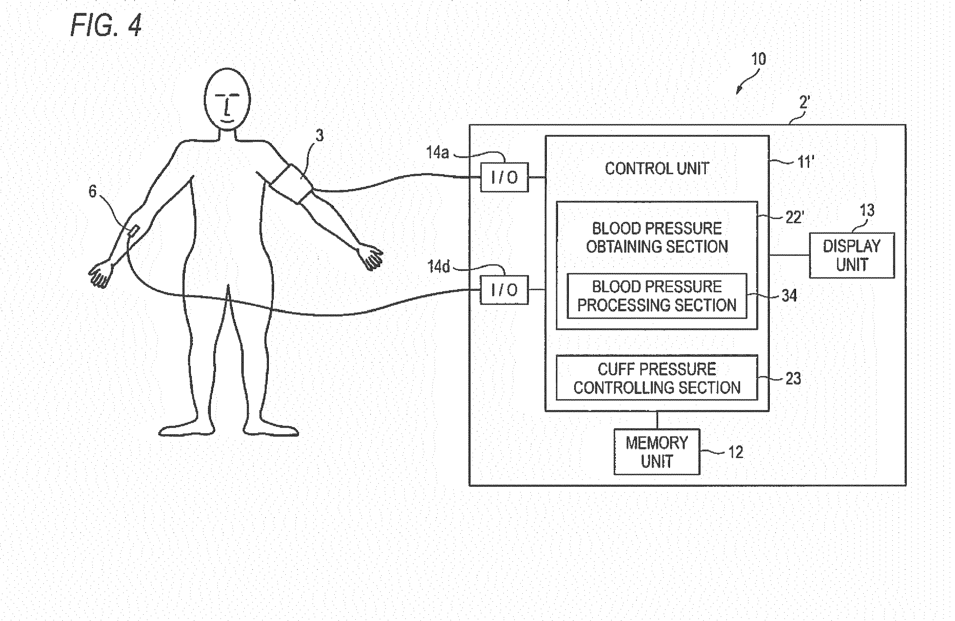

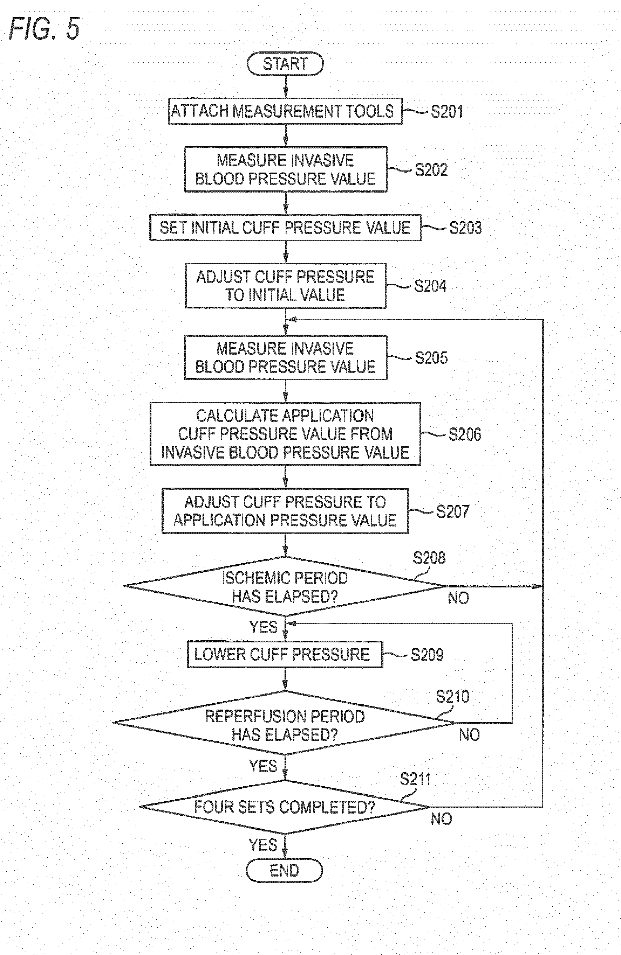

Next, a second embodiment of a bedside monitor (a bedside monitor 10) will be described with reference to Figs. 4 and 5.

As illustrated in Fig. 4, in the bedside monitor 10 of this embodiment, a blood pressure value of a patient is invasively measured by using output information of a catheter 6.

A connector of the catheter 6, which is inserted into a vessel of the right arm, is connected to an input/output port 14d.

The control unit 11' can include a blood pressure obtaining section 22' and a cuff pressure controlling section 23. The blood pressure obtaining section 22' can include a blood pressure processing section 34. The blood pressure obtaining section 22' obtains an invasive blood pressure value corresponding to a maximum blood pressure of the patient by processing vital sign obtained by the catheter 6 with the blood pressure processing section 34. Based on the invasive blood pressure value obtained by the blood pressure processing section 34, a cuff pressure to be applied to the left upper arm of the patient is controlled.

Referring to Fig. 5, a compression controlling method performed in the bedside monitor 10 will be described. It is noted that the description of processes similar to those employed in the flowchart of Fig. 2 is herein omitted. The blood pressure processing section 34 measures, based on the vital sign obtained by the catheter 6, a current invasive blood pressure value of the patient at the preliminary stage (step S202), and sets an initial cuff pressure value based on the invasive blood pressure value (step S203).

When the remote ischemic preconditioning is started, the blood pressure processing section 34 measures a current invasive blood pressure value (step S205).

Subsequently, the blood pressure processing section 34 calculates a value of a cuff pressure to be applied by a cuff 3 based on the invasive blood pressure value measured in step S205 (step S206). The value of the cuff pressure is set to a value higher than the invasive blood pressure value by 10 mmHg. The calculated cuff pressure value is fed back from the blood pressure processing section 34 to the cuff pressure controlling section 23 as data for controlling the cuff pressure.

In this manner, based on the invasive blood pressure value measured by using the catheter 6, the value of the cuff pressure to be applied by the cuff 3 is controlled. Therefore, the invasive blood pressure value changing every second can be continuously measured, so as to more precisely control the cuff pressure value. As a result, the ischemic state of the patient can be appropriately retained.

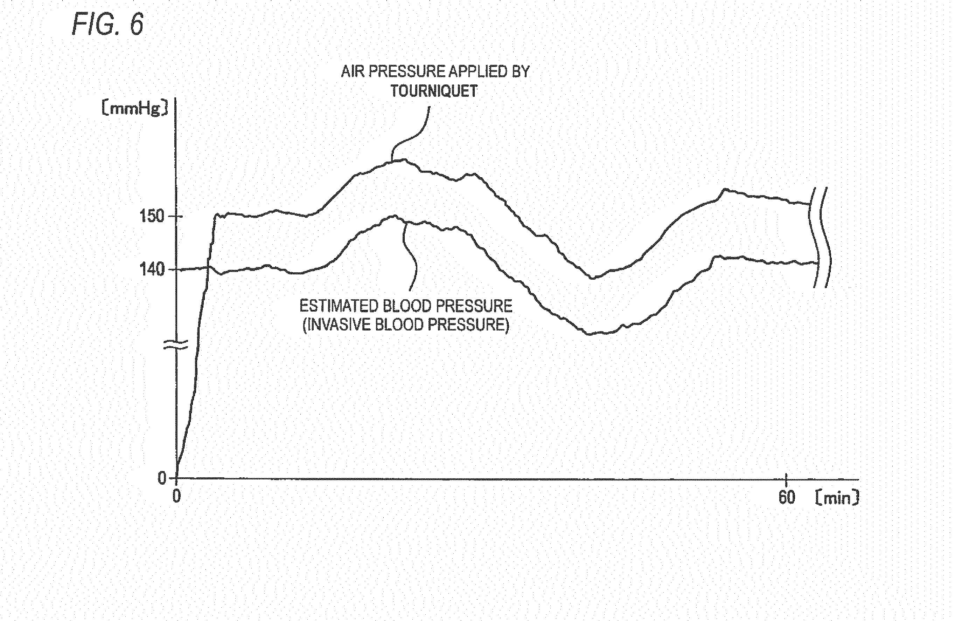

Next, a modification in which a compression pattern is changed will be described with reference to Fig. 6. In this modification, a case where an orthopedic surgery of an upper limb or a lower limb of a patient is performed will be exemplarily described.

In performing an orthopedic surgery, a compression bandage such as a tourniquet is wrapped around, for example, an upper arm or a thigh of the patient, and blood flow to a peripheral region is throttled by controlling an air pressure to be applied to the compression bandage by the cuff pressure controlling section 23 of the bedside monitor 1 or 10.

As illustrated in Fig. 6, in the application to an orthopedic surgery, an air pressure value of the tourniquet is controlled, during the surgery, at an appropriate pressure value (higher by 10 mmHg) based on a current estimated blood pressure value (or a current invasive blood pressure value) of the patient. As a result, the ischemic state of the peripheral region corresponding to a surgery target can be appropriately retained, and the amount of bleeding in a surgical field can be reduced. Besides, since the air pressure value can be automatically controlled in accordance with variation of the estimated blood pressure value (or the invasive blood pressure value), there is no need to monitor the blood pressure of the patient, and hence the burden of a healthcare professional during the surgery can be reduced so that the healthcare professional can concentrate on the surgery.

Incidentally, the present invention is not limited to the above-described embodiments but can be appropriately modified and changed. In addition, the materials, shapes, dimensions, values, forms, numbers, locations and the like of respective composing elements of the above-described embodiments are arbitrary and not specified as long as the present invention can be accomplished.

In the above description, the cuff pressure to be applied by the cuff 3 is continuously controlled from the start to the end of the remote ischemic preconditioning by using the estimated blood pressure value or the invasive blood pressure value continuously measured. The compression control is not limited to this, however, and a mean value of continuously measured estimated blood pressure values or invasive blood pressure values obtained in a prescribed time period (of, for example, 5 seconds) may be calculated to control the cuff pressure based on the mean value of the prescribed time period. When this control is employed, influence of noise or the like of equipment (such as a pulse oximeter or an electrocardiograph) on a blood pressure value can be suppressed.

Owing to the structures in the embodiments, for example, even if the blood pressure value of a patient is increased due to ischemia during a procedure, the application pressure to be applied by the cuff can be increased to an extent that the ischemic state is not removed. Besides, for example, even if the blood pressure value of the patient is lowered due to the ischemia during the procedure, the application pressure to be applied by the cuff can be lowered to an extent that the ischemic state is not removed and that an excessive load is not applied to the first region having the cuff wrapped thereon. In this manner, in accordance with the change of the blood pressure value of the patient having a part thereof in the ischemic state, the application pressure to be applied by the cuff can be controlled, so as to appropriately retain the ischemic state of the patient.

Owing to the structures in the embodiment, an estimated blood pressure value can be minimally invasively obtained by using outputs of the pulse wave obtaining section and the electrocardiogram obtaining section. Therefore, with suppressing the increase of a burden of the patient caused due to the ischemia during the procedure, the ischemic state of the patient can be appropriately retained.

Owing to the structures in the embodiments, the application pressure to be applied by the cuff can be controlled by using the invasive blood pressure value, and hence, the application pressure can be more precisely controlled.

Owing to the structures in the embodiments, influence of noise or the like of, for example, a probe or an electrode on a blood pressure value can be suppressed.

When the method in the embodiments is employed, in the same manner as in the above-described compression control device, in accordance with the change of a blood pressure value of a patient having a part thereof in an ischemic state, the application pressure applied by the cuff wrapped to the patient's body can be controlled, and hence, the ischemic state of the patient can be appropriately retained.

When the method in the embodiments is employed, the burden of a healthcare professional during the procedure of the remote ischemic preconditioning can be reduced.

When the method in the embodiments is employed, the burden of a healthcare professional during a surgery can be reduced. Besides, since no excessive pressure is applied, the burden of the patient can be reduced.

According to the compression control device and the compression control method of the embodiments, an ischemic state of a peripheral part of a patient's body can be appropriately retained.

**Claims**

1. A compression control device comprising:

    a cuff adapted to be wrapped around a first region of a living body;
    a pressure controlling section that controls an application pressure to be applied to the first region by the cuff; and
    a blood pressure obtaining section that obtains a blood pressure value from the living body,
    wherein the pressure controlling section controls the application pressure based on the blood pressure value obtained by the blood pressure obtaining section in a state where a prescribed pressure for causing an ischemic state in a part from the first region to a periphery is applied to the first region by the cuff.

2. The compression control device according to claim 1,
    wherein the blood pressure obtaining section includes:

    a pulse wave obtaining section attached to a second region of the living body different from the first region and capable of measuring a pulse wave in the second region;
    an electrocardiogram obtaining section capable of measuring an electrocardiogram of the living body; and
    a calculating section obtaining a pulse wave transit time based on the pulse wave and the electrocardiogram, and calculating an estimated blood pressure value based on the pulse wave transit time, and
    the pressure controlling section controls the application pressure based on the estimated blood pressure value in a state where the prescribed pressure for causing the ischemic state in the part from the first region to the periphery is applied to the first region by the cuff.

3. The compression control device according to claim 1,
    wherein the blood pressure obtaining section includes a catheter capable of obtaining an invasive blood pressure value from the living body, and
    the pressure controlling section controls the application pressure based on the invasive blood pressure value in a state where the prescribed pressure for causing the ischemic state in the part from the first region to the periphery is applied to the first region by the cuff.

4. The compression control device according to any one of claims 1 to 3, wherein the pressure controlling section controls the application pressure based on a mean value of blood pressure values obtained by the blood pressure obtaining section during a prescribed time period, in a state where the prescribed pressure for causing the ischemic state in the part from the first region to the periphery is applied to the first region by the cuff.

5. A compression control method for a cuff to be employed in a procedure for causing an ischemic state in a part of a living body, comprising:

    setting an initial target value of an application pressure to be applied by the cuff wrapped to a first region of the living body;
    causing the ischemic state in a part from the first region to a periphery by controlling the application pressure to be applied by the cuff based on the initial target value; and
    controlling the application pressure, in such a manner that a prescribed relationship is retained between the blood pressure value and the application pressure, based on a blood pressure value obtained from the living body while the part is in the ischemic state.

6. The compression control method according to claim 5, wherein the procedure is remote ischemic preconditioning.

7. The compression control method according to claim 5, wherein the procedure is a procedure for reducing an amount of bleeding in a surgical field during a surgery.

FIG. 1

EP 3 023 062 A1

FIG. 2

```
                    ┌─────────┐
                    │  START  │
                    └────┬────┘
                         │
          ┌──────────────▼──────────────┐
          │  ATTACH MEASUREMENT TOOLS   │──── S101
          └──────────────┬──────────────┘
                         │
          ┌──────────────▼──────────────┐
          │  MEASURE CURRENT MAXIMUM    │──── S102
          │    BLOOD PRESSURE VALUE     │
          └──────────────┬──────────────┘
                         │
          ┌──────────────▼──────────────┐
          │ SET INITIAL CUFF PRESSURE VALUE │──── S103
          └──────────────┬──────────────┘
                         │
          ┌──────────────▼──────────────┐
          │   CALIBRATE BLOOD PRESSURE  │──── S104
          │      OBTAINING SECTION      │
          └──────────────┬──────────────┘
                         │
          ┌──────────────▼──────────────┐
          │     ADJUST CUFF PRESSURE    │──── S105
          │       TO INITIAL VALUE      │
          └──────────────┬──────────────┘
                         │
          ┌──────────────▼──────────────┐
          │ OBTAIN PWTT FROM ELECTROCARDIOGRAM │──── S106
          │   AND PERIPHERAL PULSE WAVE │
          └──────────────┬──────────────┘
                         │
          ┌──────────────▼──────────────┐
          │  CALCULATE ESTIMATED BLOOD  │──── S107
          │  PRESSURE VALUE FROM PWTT   │
          └──────────────┬──────────────┘
                         │
          ┌──────────────▼──────────────┐
          │     CALCULATE APPLICATION   │──── S108
          │ CUFF PRESSURE VALUE FROM ESTIMATED │
          │     BLOOD PRESSURE VALUE    │
          └──────────────┬──────────────┘
                         │
          ┌──────────────▼──────────────┐
          │   CONTROL CUFF PRESSURE TO  │──── S109
          │ APPLICATION CUFF PRESSURE VALUE │
          └──────────────┬──────────────┘
                         │
                  S110   │
                 ◇───────▼───────◇
                 │ ISCHEMIC PERIOD │──── NO
                 │  HAS ELAPSED?   │
                 ◇───────┬───────◇
                     YES │
          ┌──────────────▼──────────────┐
          │    LOWER CUFF PRESSURE      │──── S111
          └──────────────┬──────────────┘
                         │
                  S112   │
                 ◇───────▼───────◇
                 │ REPERFUSION PERIOD │──── NO
                 │   HAS ELAPSED?    │
                 ◇───────┬───────◇
                     YES │
                  S113   │
                 ◇───────▼───────◇
                 │ FOUR SETS COMPLETED? │──── NO
                 ◇───────┬───────◇
                     YES │
                    ┌────▼────┐
                    │   END   │
                    └─────────┘
```

FIG. 3

# FIG. 4

CONTROL UNIT — 11'

BLOOD PRESSURE OBTAINING SECTION — 22'

BLOOD PRESSURE PROCESSING SECTION — 34

CUFF PRESSURE CONTROLLING SECTION — 23

DISPLAY UNIT — 13

MEMORY UNIT — 12

I/O — 14a

I/O — 14d

EP 3 023 062 A1

*FIG. 5*

```
                    ( START )
                        │
                        ▼
        ┌──────────────────────────────┐
        │  ATTACH MEASUREMENT TOOLS     ├── S201
        └──────────────────────────────┘
                        │
                        ▼
        ┌──────────────────────────────┐
        │     MEASURE INVASIVE          ├── S202
        │   BLOOD PRESSURE VALUE        │
        └──────────────────────────────┘
                        │
                        ▼
        ┌──────────────────────────────┐
        │  SET INITIAL CUFF PRESSURE VALUE ├── S203
        └──────────────────────────────┘
                        │
                        ▼
        ┌──────────────────────────────┐
        │     ADJUST CUFF PRESSURE      ├── S204
        │       TO INITIAL VALUE        │
        └──────────────────────────────┘
                        │
                        ▼
        ┌──────────────────────────────┐
        │     MEASURE INVASIVE          ├── S205
        │   BLOOD PRESSURE VALUE        │
        └──────────────────────────────┘
                        │
                        ▼
        ┌──────────────────────────────┐
        │    CALCULATE APPLICATION      │
        │  CUFF PRESSURE VALUE FROM     ├── S206
        │ INVASIVE BLOOD PRESSURE VALUE │
        └──────────────────────────────┘
                        │
                        ▼
        ┌──────────────────────────────┐
        │  ADJUST CUFF PRESSURE TO      ├── S207
        │ APPLICATION PRESSURE VALUE    │
        └──────────────────────────────┘
                        │
                        ▼                S208
              ◇ ISCHEMIC PERIOD ◇ ──── NO ──►
              ◇ HAS ELAPSED?    ◇
                     │
                    YES
                     ▼
        ┌──────────────────────────────┐
        │     LOWER CUFF PRESSURE       ├── S209
        └──────────────────────────────┘
                     │
                     ▼                S210
              ◇ REPERFUSION PERIOD ◇ ── NO ──►
              ◇ HAS ELAPSED?      ◇
                     │
                    YES
                     ▼                S211
              ◇ FOUR SETS COMPLETED? ◇ ── NO ──►
                     │
                    YES
                     ▼
                   ( END )
```

FIG. 6

FIG. 7

EP 3 023 062 A1

**EP 3 023 062 A1**

Europäisches Patentamt

European Patent Office

Office européen des brevets

# EUROPEAN SEARCH REPORT

Application Number

EP 15 19 4463

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/324429 A1 (LESCHINSKY BORIS [US]) 23 December 2010 (2010-12-23) | 1,3-7 | INV. A61B17/135 A61H9/00 A61B5/021 A61B5/0225 |
| Y | * abstract * * paragraph [0011] - paragraph [0012] * * paragraph [0054] * ----- | 2 | |
| Y | US 7 678 059 B2 (FRIEDMAN BRUCE A [US] ET AL) 16 March 2010 (2010-03-16) | 2 | |
| A | * abstract * * column 6, line 32 - column 7, line 13 * ----- | 1,3-7 | |
| A | EP 0 852 126 A2 (NIHON KOHDEN CORP [JP]) 8 July 1998 (1998-07-08) * abstract; figure 1 * ----- | 1-7 | |
| | | | TECHNICAL FIELDS SEARCHED (IPC) A61B A61H |

The present search report has been drawn up for all claims

| Place of search | Date of completion of the search | Examiner |
|---|---|---|
| The Hague | 17 March 2016 | Van Dop, Erik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 15 19 4463

This annex lists the patent family members relating to the patent documents cited in the above-mentioned European search report.
The members are as contained in the European Patent Office EDP file on
The European Patent Office is in no way liable for these particulars which are merely given for the purpose of information.

17-03-2016

| Patent document cited in search report | | Publication date | Patent family member(s) | | Publication date |
|---|---|---|---|---|---|
| US 2010324429 | A1 | 23-12-2010 | EP | 2448474 A2 | 09-05-2012 |
| | | | US | 2010324429 A1 | 23-12-2010 |
| | | | US | 2012130419 A1 | 24-05-2012 |
| | | | US | 2014296757 A1 | 02-10-2014 |
| | | | WO | 2011005538 A2 | 13-01-2011 |
| US 7678059 | B2 | 16-03-2010 | DE | 102006047491 A1 | 26-04-2007 |
| | | | US | 2007106163 A1 | 10-05-2007 |
| EP 0852126 | A2 | 08-07-1998 | DE | 69728849 D1 | 03-06-2004 |
| | | | DE | 69728849 T2 | 02-09-2004 |
| | | | EP | 0852126 A2 | 08-07-1998 |
| | | | JP | 3581975 B2 | 27-10-2004 |
| | | | JP | H10192247 A | 28-07-1998 |
| | | | US | 5876348 A | 02-03-1999 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5323719 B